# FASCICULE DE BREVET EUROPEEN

(11) **EP 1 249 225 B1**
(45) Date de publication et mention de la délivrance du brevet: **11.10.2006**
(21) Numéro de dépôt: 02290884.2
(22) Date de dépôt: 09.04.2002
(51) Int. Cl.: A61K 8/04, A61K 8/73, A61K 8/81, A61Q 1/00

(54) **Mascara comprenant un polymère acrylique**
Mascara enthaltend einen Acrylatpolymer
Mascara containing an acrylic polymer

(30) Priorité: 10.04.2001 FR 0104886
(43) Date de publication de la demande: 16.10.2002
(73) Titulaire: L'ORÉAL, 75008 Paris (FR)
(72) Inventeur: De La Poterie, Valérie, 77820 Le Chatelet-en-Brie (FR)
(74) Mandataire: Kromer, Christophe

(56) Documents cités:
- EP-A- 0 745 372
- EP-A- 0 875 243
- EP-A- 1 018 334
- EP-A- 1 066 819
- EP-A- 1 101 488
- EP-A- 1 163 898
- WO-A-96/33690
- US-A- 4 423 031

## Description

La présente invention a pour objet une composition de mascara, destinée au maquillage des cils et/ou des sourcils d'êtres humains, comprenant une dispersion aqueuse de polymère acrylique. L'invention se rapporte également à un procédé de maquillage des cils et/ou des sourcils.

Les mascaras sont couramment préparés selon deux types de formulations à base de cires : les mascaras aqueux, dits mascaras crèmes, sous forme d'émulsion de cires dans l'eau ; les mascaras anhydres ou à faible teneur en eau, dits mascaras waterproofs, sous forme de dispersion de cires dans des solvants organiques volatils.

Il est également connu d'employer avec les cires des polymères filmogènes qui peuvent être solubilisés ou dispersés dans un milieu aqueux, comme notamment décrit dans les documents FR-A-2528699 et EP-A-655234.

Or le film de maquillage obtenu après l'application de ces mascaras ne présente pas une cohésion suffisante car les cires fragilisent le film. Ce film n'est pas suffisamment résistant aux frottements, notamment des doigts, et/ou à l'eau, lors de baignades ou de douches par exemple, ou bien encore aux larmes ou à la sueur. Le mascara a alors tendance à s'effriter dans le temps : des grains se déposent et laissent des traces autour de l'oeil. Le maquillage n'étant pas suffisamment résistant, il présente une mauvaise tenue dans le temps et ne peut pas être gardé plus d'une journée. L'utilisatrice doit donc appliquer le mascara chaque jour pour garder des cils bien maquillés.

Par ailleurs, certaines utilisatrices souhaitent avoir des cils bien colorés sans les épaissir. Il est possible de colorer les cils par l'emploi d'une teinture utilisant des colorants, similaire à une coloration capillaire. Toutefois, ce type de teinture se fait généralement à l'institut de beauté en raison de la technique particulière nécessaire à mettre en oeuvre, ce qui représente une certaine contrainte pour l'utilisatrice. Il est donc souhaitable pour l'utilisatrice de pouvoir se colorer facilement les cils sans les épaissir en utilisant un produit différent d'une teinture.

La présente invention a donc pour but de proposer une composition de mascara conduisant à un maquillage naturel présentant une bonne tenue dans le temps, supérieure à une journée, voire deux journées ou plus.

Le demandeur a constaté qu'un tel mascara pouvait être obtenu avec une composition exempte de cire et comprenant un polymère acrylique filmogène particulier en dispersion aqueuse. La composition est facile à appliquer et gaine bien les cils ; après séchage, la composition forme un dépôt lisse et homogène. Le maquillage est peu chargeant, c'est à dire qu'il n'épaissit pas les cils : on obtient ainsi un maquillage naturel. Le maquillage est également confortable et présente une bonne tenue dans le temps : le film ne s'effrite pas et le maquillage déposé sur les cils est conservé plus d'une journée, voire même plus de deux journées.

De façon plus précise, l'invention a pour objet une composition de mascara exempte de cire comprenant, dans un milieu aqueux cosmétiquement acceptable, un polymère acrylique filmogène sous forme de particules solides dispersées dans le milieu aqueux, ledit polymère filmogène acrylique étant apte à former un film ayant une reprise en eau, après 10 minutes d'immersion dans l'eau à 25 °C, inférieure à 25 % en poids, la composition ayant une viscosité, mesurée à 25 °C, à une vitesse de cisaillement de 200 s⁻¹, allant de 4 Pa.s à 18 Pa.s.

L'invention a aussi pour objet un procédé de maquillage des cils et/ou des sourcils, caractérisé par le fait que l'on applique sur les cils et/ou les sourcils une composition de maquillage telle que définie précédemment.

L'invention a également pour objet l'utilisation d'une composition de maquillage telle que définie précédemment pour l'obtention d'un maquillage des cils et/ou des sourcils présentant une tenue supérieure à un jour, notamment supérieure à deux jours, et/ou naturel.

L'invention a encore pour objet l'utilisation d'un polymère acrylique filmogène tel que défini précédemment, dans une composition de mascara exempte de cire, ayant une viscosité, mesurée à 25 °C, à une vitesse de cisaillement de 200 s⁻¹, allant de 4 Pa.s à 18 Pa.s, pour obtenir un maquillage des cils ou des sourcils présentant une tenue supérieure à un jour, notamment supérieure à deux jours, et/ou naturel.

Par " cire", on entend dans la présente demande un composé gras lipophile, solide à température ambiante (25°C) et pression atmosphérique (760 mm de Hg, soit 10⁵ Pa), à changement d'état solide/liquide réversible, ayant une température de fusion allant de 30 °C à 200 °C.
En portant la cire à sa température de fusion, il est possible de la rendre miscible aux huiles et de former un mélange homogène microscopiquement, mais en ramenant la température du mélange à la température ambiante, on obtient une recristallisation de la cire dans les huiles du mélange.
Les valeurs de point de fusion correspondent au pic de fusion mesurée à l'aide d'un calorimètre à balayage différentiel (D.S.C.), par exemple le calorimètre vendu sous la dénomination DSC 30 par la société METLER, avec une montée en température de 5 ou 10 °C par minute.

Ainsi, la composition selon l'invention ne contient pas de cire telle que définie précédemment.

Le polymère acrylique présent dans la composition permet d'obtenir un produit de maquillage présentant une bonne tenue dans le temps, notamment une tenue supérieure à un jour, voire même deux à trois jours, et une bonne résistance à l'eau.

Selon la présente demande, on entend par "reprise en eau du polymère acrylique", le pourcentage en poids d'eau absorbé par le polymère acrylique après 10 minutes d'immersion dans l'eau, à 25 °C. La reprise en eau est mesurée pour une couche de 300 µm d'épaisseur (avant séchage) déposée sur une plaque puis séchée pendant 24 heures à 30 °C et à 50 % d'humidité relative ; des morceaux d'environ 1 cm² découpés dans le film sec sont pesés (mesure de la masse M1) puis immergés dans l'eau pendant 10 minutes ; après immersion, le morceau de film est essuyé pour éliminer l'excédent d'eau en surface puis pesé (mesure de la masse M2). La différence M2 - M1 correspond à la quantité d'eau absorbée par le polymère.
La reprise en eau est égale à [ (M2- M1) / M1 ] x 100 et est exprimée en pourcentage de poids d'eau par rapport au poids de polymère.

De préférence, ledit polymère filmogène acrylique est apte à former un film ayant une reprise en eau, après 10 minutes d'immersion dans l'eau à 25 °C, inférieure à 20 % en poids, et mieux inférieure à 15 % en poids.

Avantageusement, le polymère acrylique est apte à former un film ayant une dureté allant de 8 à 40 secondes, de préférence de 10 à 35 secondes, préférentiellement de 10 à 30 secondes, mieux de 15 à 30 secondes et encore mieux de 20 à 30 secondes.

La dureté du film de polymère est mesurée sur un film obtenu après séchage, durant 24 heures à 30 °C et à 50 % d'humidité relative, d'une couche de 300 µm d'épaisseur (avant séchage) d'une dispersion aqueuse à 40 % de matière sèche desdites particules de polymère radicalaire. La dureté du film est mesurée selon la norme ASTM D-43-66, ou la norme NF-T 30-016 (octobre 1981), à l'aide d'un pendule de Persoz.
Le polymère acrylique utilisé dans la composition selon l'invention peut présenter à lui seul les propriétés de reprise en eau et/ou de dureté définies précédemment. Il peut également présenter ces propriétés en présence d'agent auxiliaire de filmification choisis parmi les agents plastifiants et/ou les agents de coalescence bien connus de l'homme du métier. De préférence, le polymère acrylique à lui seul satisfait à ces propriétés de résistance à l'eau et/ou de dureté.

Les particules solides de polymère acrylique dispersées dans le milieu aqueux de la composition ont généralement une taille pouvant aller de 10 nm à 200 nm, de préférence allant de 20 nm à 150 nm, et mieux allant de 50 nm à 100 nm.

Les polymères filmogènes acryliques utilisables selon l'invention peuvent résulter de la polymérisation d'au moins un monomère à insaturation éthylénique choisis parmi les acides carboxyliques α,β-éthyléniques, leurs esters et leurs amides.

Comme acide carboxylique insaturé α,β-éthyléniques, on peut utiliser l'acide acrylique, l'acide méthacrylique, l'acide crotonique, l'acide maléique, l'acide itaconique. On utilise de préférence l'acide (méth)acrylique et l'acide crotonique, et plus préférentiellement l'acide (méth)acrylique.

Les esters de ces acides carboxyliques peuvent être choisis parmi les esters de l'acide (méth)acrylique (encore appelé les (méth)acrylates), notamment des (méth)acrylates d'alkyle, en particulier d'alkyle en C₁-C₃₀, de préférence en C₁-C₂₀, des (méth)acrylates d'aryle, en particulier d'aryle en C₆-C₁₀, des (méth)acrylates d'hydroxyalkyle, en particulier d'hydroxyalkyle en C₂-C₆.
Parmi les (méth)acrylates d'alkyle, on peut citer le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle.
Parmi les (méth)acrylates d'hydroxyalkyle, on peut citer l'acrylate d'hydroxyéthyle, l'acrylate de 2-hydroxypropyle, le méthacrylate d'hydroxyéthyle, le méthacrylate de 2-hydroxypropyle.
Parmi les (méth)acrylates d'aryle, on peut citer l'acrylate de benzyle et l'acrylate de phényle.

Il est possible bien entendu d'employer un mélange de ces monomères.

Les esters de l'acide (méth)acrylique particulièrement préférés sont les (méth)acrylates d'alkyle.

Selon la présente invention, le groupement alkyle des esters peut être soit fluoré, soit perfluoré, c'est-à-dire qu'une partie ou la totalité des atomes d'hydrogène du groupement alkyle sont substitués par des atomes de fluor.

Comme amides desdits acides caboxyliques, on peut par exemple citer les (méth)acrylamides, et notamment les N-alkyl (méth)acrylamides, en particulier d'alkyl en C₂-C₁₂. Parmi les N-alkyl (méth)acrylamides, on peut citer le N-éthyl acrylamide, le N-t-butyl acrylamide, le N-t-octyl acrylamide et le N-undécylacrylamide.

Le polymère filmogène acrylique utilisable sein l'invention peut comprendre, en plus des monomères cités précédement, au moins un monomère styrénique, tel que le styrène ou l'alpha-méthyl styrène.

Comme polymère acrylique, on peut utiliser ceux vendus sous les dénominations "SYNTRAN® 5190 », « SYNTRAN® 5760 », « SYNTRAN ®5009» par la société INERPOLYMER, « DOW LATEX 424® » par la société DOW CHEMICAL.

Le polymère acrylique sous forme de particules en dipsersion aqueuse peut être présent dans la composition du produit selon l'invention en une teneur, en poids de matières sèches, allant de 5 % à 60 % en poids, par rapport au poids total de la composition, de préférence de 10 % à 45 % en poids, mieux de 15 % à 35 % en poids, et préférentiellement de 20 % à 35 % en poids.

Le milieu aqueux de la composition peut être constitué essentiellement d'eau. Il peut comprendre également un mélange d'eau et de solvant miscible à l'eau comme les monoalcools inférieurs ayant de 1 à 5 atomes de carbone, les cétones en C₃-C₄, les aldéhydes en C₃-C₄. Comme solvant miscible à l'eau, on peut de préférence utiliser de l'éthanol. La teneur en solvant miscible à l'eau peut aller de 0,1 % à 15 % en poids, et mieux de 1 à 8 % en poids, par rapport au poids total de la composition.

Avantageusement, la composition peut avoir une viscosité allant de 5 Pa.s (60 poises) à 15 Pa.s (150 poises), et mieux de 6 Pa.s (70 poises) à 12 Pa.s (120 poises). Une telle viscosité permet une application facile et rapide de la composition, ainsi qu'un gainage régulier sur toute la longueur des cils. La viscosité est mesurée à 25 °C avec un viscosimètre RHEOMAT RM 180 équipé d'un mobile n°4, la mesure étant effectuée après 10 minutes de rotation du mobile (temps au bout duquel on observe une stabilisation de la viscosité et de la vitesse de rotation du mobile), à un cisaillement de 200 s⁻¹.

Pour conférer à la composition selon l'invention la viscosité requise pour l'application sur les fibres kératiniques, et notamment les cils, la composition peut comprendre un agent épaississant permettant d'ajuster la viscosité souhaitée.

Parmi les agents épaississants utilisables selon l'invention, on peut citer :
- les épaississants cellulosiques hydrosoluble tels que l'hydroxyéthylcellulose, la méthylcellulose, l'hydroxypropylcellulose et la carboxyméthylcellulose. Parmi celles-ci, on peut citer notamment les gommes vendues sous la dénomination de "Cellosize QP 4400 H" par la Société Amercol,
- la gomme de guar, notamment celles vendues sous la dénomination VIDOGUM GH 175 par la société UNIPECTINE et sous la dénomination JAGUAR C par la société MEYHALL,
- la gomme de guar quaternisée vendue sous la dénomination de "Jaguar C-13-S" par la Société Meyhall,
- les gommes de guar non-ioniques comprenant des groupements hydroxyalkyle en C₁-C₆. On peut mentionner à titre d'exemple, les groupements hydroxyméthyle, hydroxyéthyle, hydroxypropyle et hydroxybutyle. De telles gommes de guar sont notamment vendues sous les dénominations commerciales JAGUAR HP8, JAGUAR HP60 et JAGUAR HP120 et JAGUAR HP 105 par la société MEYHALL, ou sous la dénomination GALACTASOL 40H4FD2 par la société AQUALON.
- les gommes de xanthane, de caroube, de scléroglucane, de gellane, de rhamsan, de karoya,
- les alginates, la maltodextrine, l'amidon et ses dérivés, l'acide hyaluronique et ses sels,
- les argiles, et notamment les montmorillonites, les hectorites, les laponites
- les acides polyacryliques réticulés tels que les "Carbopol" de la Société Goodrich,
- les polymères poly(métha)crylates de glycéryle vendus sous les dénominations de "Hispagel" ou "Lubragel" par les Sociétés Hispano Quimica ou Guardian,
- la polyvinylpyrrolidone,
- l'alcool polyvinylique,
- les polymères et les copolymères réticulés d'acrylamide, tels que ceux vendus sous les dénominations de "PAS 5161" ou "Bozepol C" par la Société Hoechst, de "Sepigel 305" par la Société Seppic par la Société Allied Colloïd, ou encore
- les homopolymères réticulés de chlorure de méthacryloyloxyéthyltriméthylammonium vendus sous la dénomination de "Salcare SC95" par la Société Allied Colloïd.
- les polymères associatifs et notamment les polymère acryliques associatifs.

Dans la composition selon l'invention, l'agent épaississant peut être présent une quantité efficace pour que la composition présente la viscosité telle que définie précédemment. La teneur en agent épaississant peut par exemple aller de 0,1 % à 10 % en poids, par rapport au poids total de la composition, et de préférence de 0,5 % à 5 % en poids.

La composition peut comprendre en outre d'autres ingrédients habituellement utilisés en cosmétique. De tels ingrédients peuvent être notamment choisis parmi les agents plastifiants, les agents de coalescence, les charges, les matières colorantes comme les pigments ou les colorants, les tensio-actifs, les conservateurs, les huiles, les agents cosmétiques comme les agents hydratants et les agents anti-UV qui sont bien connus de l'état de la technique. Bien entendu, l'homme du métier veillera à choisir ce ou ces éventuels additifs et/ou leur quantité, de manière telle que les propriétés avantageuses du maquillage soient conservées.

Comme charge, on peut notamment utiliser les charges habituellement employées dans les compositions de mascaras. On peut par exemple utiliser la silice pyrogénée, l'amidon, comme l'amidon de riz, le talc ou le polytétrafluoroéthylène.
Les charges peuvent être présentes dans la composition en une teneur allant de 0,1 % à 6 % en poids, par rapport au poids total de la composition.

La composition peut également comprend un polymère filmogène auxiliaire, pour permettre de modifier les propriétés cosmétiques et physico-chimiques du film de maquillage. Ce polymère filmogène auxiliaire peut être sous forme dissous ou bien sous forme de particules solides dispersées dans le milieu aqueux de la composition.
Comme polymère filmogène auxiliaire, on peut par exemple citer les polymères polycondensats comme les polyuréthanes ou bien encore les polyesters comme les polyesters à groupement sulfonique.

Selon un mode préféré de réalisation de l'invention, la composition de mascara ne contient comme polymère filmogène sous forme de dispersion aqueuse un ou plusieurs polymère(s) acrylique(s) tels que défini(s) précédemment.

La composition de maquillage selon l'invention peut être conditionnée dans un ensemble d'application comprenant un réservoir, un moyen amovible pour fermer, de préférence de manière étanche, ledit réservoir, et un organe d'application de la composition sur les cils et/ou les sourcils.

Aussi, l'invention a également pour objet un produit de mascara, comprenant un réservoir contenant une composition de mascara telle que décrite précédemment, un organe d'application de la composition sur les cils et/ou les sourcils et un moyen amovible de fermeture destiné à fermer le récipient.

Ledit organe d'application permet le prélèvement de la composition et la restitution de la composition prélevée sur les cils. Cet organe d'application est de préférence solidaire des moyens de fermeture étanche de l'ensemble.
L'ensemble d'application peut également comprendre un organe d'essorage (ou essoreur) dudit organe d'application, l'organe d'essorage pouvant être solidaire du réservoir.

L'organe d'application peut être de préférence une brosse à mascara bien connue de l'homme du métier. Une telle brosse comprend notamment des poils disposés radialement autour d'une âme torsadée, en particulier une âme métallique. La brosse peut être de forme variée et comporter des découpes. Des brosses à mascara sont par exemple décrites dans les documents FR-A-2607373,
EP-A-611170, EP-A-811336, EP-A-811337, EP-A-842620, dont le contenu est incorporé dans la présente demande à titre de référence.

Dans un mode de réalisation, l'ensemble d'application peut comporter un réservoir contenant une composition de mascara telle que définie précédemment, muni d'un goulot et d'un applicateur. L'applicateur peut comporter une tige munie à une première extrémité d'un organe d'application et solidaire à une deuxième extrémité d'un élément de préhension constituant un moyen de fermeture de l'ensemble d'application.

L'ensemble d'application peut comporter, en outre, un essoreur annulaire fixé dans le goulot du réservoir et traversé par la tige, cet essoreur étant apte à essorer cette tige et/ou l'organe d'application. Avantageusement, cet essoreur a la forme d'un doigt de gant pourvu d'un orifice de passage central qui peut être éventuellement floqué. Un tel dispositif est notamment décrit dans le document FR-A-2705876.

L'invention est illustrée plus en détail dans les exemples suivants.

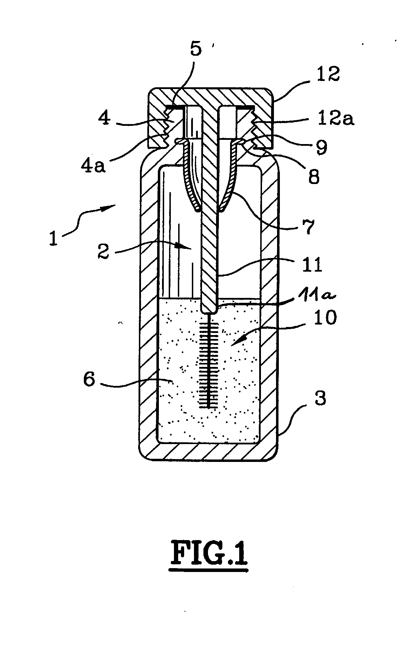

La figure 1 est une vue en élévation d'un ensemble de mascara conforme à l'invention.

En se reportant à la figure 1, on peut voir un ensemble d'application 1 de mascara comprenant un applicateur 2 et un réservoir 3, muni d'un goulot 4 fileté surmonté d'un joint d'étanchéité 5, contenant une composition de mascara 6 ayant la composition de l'exemple 1. Le réservoir 3 comporte dans son goulot 4 un essoreur 7 maintenu en place par un bourrelet 8 qui coopère avec une gorge 9 logée aussi dans le goulot 4. L'essoreur est constitué, de façon connue, en une matière souple et élastique.

L'applicateur 2 comprend un organe d'application 10 fixé à une extrémité 11a d'une tige 11. Un moyen de préhension 12 est solidaire de l'extrémité de la tige 11 opposée à l'organe d'application 10. L'organe d'application 10 est une brosse à mascara comprenant de façon connue des poils répartis radialement en nappe hélicoïdale autour d'une âme torsadée.

Le moyen de préhension 12 formant un capuchon comporte un filetage 12a qui coopère avec le filetage 4a du goulot 4 du réservoir 3. L'obturation étanche du réservoir 3 est obtenue en vissant le moyen de préhension 10 sur le goulot 4 du réservoir muni du joint 5.

Des exemples de compositions de mascara, pouvant être conditionnées dans l'ensemble d'application décrit précédemment, sont donnés ci-après.

### Exemples de composition :

### Exemple 1 :

On a préparé un mascara ayant la composition suivante :
- dispersion aqueuse de polymère acrylique
   à 40 % de matières sèches
   (SYNTRAN® 5190 de INTERPOLYMERE) 20,5 g MA
- Hydroxyéthyl cellulose
   (Cellosize QP 4400 H d'AMERCHOL) 1,9 g
- Silice pyrogénée (AEROSIL 200 de DEGUSSA) 1 g
- éthanol 5 g
- propylène glycol 5 g
- pigments 5 g
- conservateurs qs
- eau qsp 100 g

La dispersion aqueuse de polymère acrylique « SYNTRAN® 5190 » forme un film ayant une reprise en eau à 25 °C, après 10 minutes d'immersion dans l'eau, égale à 8 % en poids, les mesures étant effectuées selon les protocoles décrit précédemment.

La mascara s'applique facilement sur les cils et conduit à un maquillage présentant une bonne tenue pendant au moins un jour, voire deux à 4 jours, résistant aux frottements des doigts.

### Exemple 2 :

On a préparé un mascara ayant la composition suivante :
- dispersion aqueuse de polymère acrylique
   à 40 % de matières sèches
   (SYNTRAN® 5760 de INTERPOLYMERE) 30 g MA
- Hydroxyéthyl cellulose
   (Cellosize QP 4400 H d'AMERCHOL) 1,9 g
- éthanol 5 g
- propylène glycol 5 g
- pigments 5 g
- conservateurs qs
- eau qsp 100 g

La dispersion aqueuse de polymère acrylique « SYNTRAN® 5760 » forme un film ayant une reprise en eau à 25 °C, après 10 minutes d'immersion dans l'eau, égale à 10 % en poids, les mesures étant effectuées selon les protocoles décrit précédemment.

Le mascara s'applique facilement et adhère bien sur les cils. Le film de maquillage gaine bien les cils sur toute leur longueur et présente une bonne tenue dans le temps, supérieure à deux jours.

## Revendications

1. Composition de mascara exempte de cire comprenant, dans un milieu aqueux cosmétiquement acceptable, un polymère acrylique filmogène sous forme de particules solides dispersées dans le milieu aqueux, ledit polymère filmogène acrylique étant apte à former un film ayant une reprise en eau, après 10 minutes d'immersion dans l'eau à 25 °C, inférieure à 25 % en poids, la composition ayant une viscosité, mesurée à 25 °C, à une vitesse de cisaillement de 200 s⁻¹, allant de 4 Pa.s à 18 Pa.s.

2. Composition de mascara selon la revendication 1, **caractérisée par le fait que** le polymère acrylique est apte à former un film ayant une reprise en eau inférieure à 20 % en poids.

3. Composition de mascara selon la revendication 1 ou 2, **caractérisée par le fait que** le polymère acrylique est apte à former un film ayant une reprise en eau inférieure à 15 % en poids.

4. Composition de mascara selon l'une quelconque des revendications précédentes, **caractérisée par le fait que** le polymère acrylique est apte à former un film ayant une dureté ; mesurée selon la norme NF-T- 30-016 à l'aide d'un pendule de Persoz, allant de 8 à 40 secondes.

5. Composition de mascara selon la revendication 4, **caractérisée par le fait que** le polymère acrylique est apte à former un film ayant une dureté allant de 10 à 35 secondes.

6. Composition selon la revendication 4 ou 5, **caractérisée par le fait que** le polymère acrylique est apte à former un film ayant une dureté allant de 10 à 30 secondes.

7. Composition selon l'une quelconque des revendications 4 à 6, **caractérisée par le fait que** le polymère acrylique est apte à former un film ayant une dureté allant de 15 à 30 secondes.

8. Composition selon l'une quelconque des revendications précédentes, **caractérisée par le fait que** les particules de polymère acrylique ont une taille allant de 10 nm à 200 nm.

9. Composition selon l'une quelconque des revendications précédentes, **caractérisée par le fait que** les particules de polymère acrylique ont une taille allant de 20 nm à 150 nm.

10. Composition selon l'une quelconque des revendications précédentes, **caractérisée par le fait que** les particules de polymère acrylique ont une taille allant de 50 nm à 100 nm.

11. Composition de mascara selon l'une quelconque des revendications précédentes, **caractérisée par le fait que** le polymère acrylique résulte de la polymérisation d'au moins un monomère à insaturation éthylénique choisis parmi les acides carboxyliques α,β-éthyléniques, leurs esters et leurs amides.

12. Composition de mascara selon la revendication 11, **caractérisée par le fait que** l'acide carboxylique insaturé α,β-éthylénique est choisi parmi l'acide acrylique, l'acide méthacrylique, l'acide crotonique, l'acide maléique, l'acide itaconique.

13. Composition de mascara selon la revendication 11 ou 12, **caractérisée par le fait que** l'acide carboxylique insaturé α,β-éthyléniques est l'acide (méth)acrylique.

14. Composition de mascara selon la revendication 13, **caractérisée par le fait que** l'ester de l'acide carboxylique insaturé α,β-éthylénique est choisi parmi les esters de l'acide (méth)acrylique.

15. Composition de mascara selon la revendication 14, **caractérisée par le fait que** ledit ester est choisi parmi les (méth)acrylates d'alkyle avec un radical alkyle en C₁-C₃₀, les (méth)acrylates d'aryle avec un radical aryle en C₆-C₁₀, les (méth)acrylates d'hydroxyalkyle avec un radical hydroxyalkyle en C₂-C₆.

16. Composition de mascara selon l'une quelconque des revendications 11 à 15, **caractérisée par le fait que** l'ester d'acide carboxylique insaturé α,β-éthylénique est choisi parmi le méthacrylate de méthyle, le méthacrylate d'éthyle, le méthacrylate de butyle, le méthacrylate d'isobutyle, le méthacrylate d'éthyl-2 hexyle, le méthacrylate de lauryle, le méthacrylate de cyclohexyle, l'acrylate d'hydroxyéthyle, l'acrylate de 2-hydroxypropyle, le méthacrylate d'hydroxyéthyle, le méthacrylate de 2-hydroxypropyle, l'acrylate de benzyle, l'acrylate de phényle, et leurs mélanges.

17. Composition de mascara selon l'une quelconque des revendications 11 à 16, **caractérisée par le fait que** l'amide de l'acide carboxylique insaturé α,β-éthylénique est choisi parmi les N-alkyl (C₂-C₁₂) (méth)acrylamides, tels que le N-éthyl acrylamide, le N-t-butyl acrylamide, le N-t-octyl acrylamide, le N-undécylacrylamide.

18. Composition de mascara selon l'une quelconque des revendications 11 à 17, **caractérisée par le fait que** le polymère filmogène acrylique résulte également de la polymérisation d'au moins un monomère styrénique tels que le styrène ou l'alpha-méthyl styrène.

19. Composition de mascara selon l'une quelconque des revendications précédentes, **caractérisée par le fait que** le polymère acrylique est présent en une teneur, en poids de matières sèches, allant de 5 % à 60 % en poids, par rapport au poids total de la composition.

20. Composition de mascara selon l'une quelconque des revendications précédentes, **caractérisée par le fait que** le polymère acrylique est présent en une teneur, en poids de matières sèches, allant de 10 % à 45 % en poids, par rapport au poids total de la composition.

21. Composition de mascara selon l'une quelconque des revendications précédentes, **caractérisée par le fait que** le polymère acrylique est présent en une teneur, en poids de matières sèches, allant de 15 % à 35 % en poids, par rapport au poids total de la composition.

22. Composition de mascara selon l'une quelconque des revendications précédentes, **caractérisée par le fait que** la viscosité de la composition va de 5 Pa.s à 15 Pa.s, et mieux de 6 Pa.s à 12 Pa.s.

23. Composition de mascara selon l'une quelconque des revendications précédentes, **caractérisée par le fait que** la composition comprend au moins un agent épaississant.

24. Composition de mascara selon l'une quelconque des revendications précédentes, **caractérisée par le fait que** la composition comprend au moins un agent épaississant choisi dans le groupe formé par les épaississants cellulosiques, les gommes de guar, les gommes de xanthane, de caroube, de scléroglucane, de gellane, de rhamsan, de karoya, les alginates, la maltodextrine, l'amidon et ses dérivés, l'acide hyaluronique et ses sels, les argiles, les acides polyacryliques réticulés, les poly(métha)crylates de glycéryle, la polyvinylpyrrolidone, l'alcool polyvinylique, les polymères et les copolymères réticulés d'acrylamide, les homopolymères réticulés de chlorure de méthacryloyloxyéthyltriméthylammonium, les polymères associatifs.

25. Composition de mascara selon l'une quelconque des revendications précédentes, **caractérisée par le fait que** la composition comprend en outre au moins un ingrédient choisi dans le groupe formé par les agents plastifiants, les agents de coalescence, les charges, les matières colorantes, les tensio-actifs, les conservateurs, les huiles, les agents cosmétiques.

26. Composition de mascara selon l'une quelconque des revendications précédentes, **caractérisée par le fait que** la composition comprend en outre au moins une charge choisie dans le groupe formé par la silice pyrogénée, l'amidon, le talc et le polytétrafluoroéthylène.

27. Composition de mascara selon la revendication 25 ou 26, **caractérisée par le fait que** la charge est présente en une teneur allant de 0,1 % à 6 % en poids, par rapport au poids total de la composition.

28. Composition de mascara selon l'une quelconque des revendications précédentes, **caractérisé par le fait que** la composition comprend, en outre, un polymère filmogène auxiliaire.

29. Produit de mascara, comprenant un réservoir (3) contenant une composition de mascara, notamment des cils, un moyen amovible de fermeture (12) destiné à fermer le réservoir et un organe d'application (10) de la composition sur lesdites fibres, **caractérisé par le fait que** la composition de mascara est conforme selon l'une quelconque des revendications précédentes.

30. Produit de mascara selon la revendication 29, **caractérisé par le fai**t que l'organe d'application est solidaire du moyen amovible de fermeture (12).

31. Produit de mascara selon l'une des revendications 29 ou 30, **caractérisé par le fait que** l'organe d'application (10) est une brosse à mascara.

32. Produit de mascara selon l'une quelconque des revendications 29 à 31, **caractérisé par le fait qu**'il comprend en outre un organe d'essorage (7).

33. Procédé de maquillage des cils et/ou des sourcils, **caractérisé par le fait que** l'on applique sur les cils et/ou les sourcils une composition de mascara selon l'une quelconque des revendications 1 à 28.

34. Utilisation d'une composition de maquillage selon l'une quelconque des revendications 1 à 28 pour l'obtention d'un maquillage des cils et/ou des sourcils présentant une tenue supérieure à un jour, notamment supérieure à deux jours, et/ou naturel.

35. Utilisation d'un polymère acrylique filmogène sous forme de particules solides dispersées dans le milieu aqueux, ledit polymère filmogène acrylique étant apte à former un film ayant une reprise en eau, après 10 minutes d'immersion dans l'eau à 25 °C, inférieure à 25 % en poids, dans une composition de mascara exempte de cire ayant une viscosité, mesurée à 25 °C, à une vitesse de cisaillement de 200 s⁻¹, allant de 4 Pa.s à 18 Pa.s, pour obtenir un maquillage des cils ou des sourcils présentant une tenue supérieure à un jour, notamment supérieure à deux jours, et/ou naturel.

## Claims

1. Wax-free mascara composition comprising, in a cosmetically acceptable aqueous medium, a film-forming acrylic polymer in the form of solid particles dispersed in the aqueous medium, the said film-forming acrylic polymer being capable of forming a film having a water uptake, after immersion for 10 minutes in water at 25°C, of less than 25% by weight, the composition having a viscosity, measured at 25°C, at a shear rate of 200 s⁻¹, ranging from 4 Pa.s to 18 Pa.s.

2. Mascara composition according to Claim 1, **characterized in that** the acrylic polymer is capable of forming a film having a water uptake of less than 20% by weight.

3. Mascara composition according to Claim 1 or 2, **characterized in that** the acrylic polymer is capable of forming a film having a water uptake of less than 15% by weight.

4. Mascara composition according to any one of the preceding claims, **characterized in that** the acrylic polymer is capable of forming a film having a hardness, measured according to standard NF-T-30-016 using a Persoz pendulum, ranging from 8 to 40 seconds.

5. Mascara composition according to Claim 4, **characterized in that** the acrylic polymer is capable of forming a film having a hardness ranging from 10 to 35 seconds.

6. Composition according to Claim 4 or 5, **characterized in that** the acrylic polymer is capable of forming a film having a hardness ranging from 10 to 30 seconds.

7. Composition according to any one of Claims 4 to 6, **characterized in that** the acrylic polymer is capable of forming a film having a hardness ranging from 15 to 30 seconds.

8. Composition according to any one of the preceding claims, **characterized in that** the acrylic polymer particles range from 10 nm to 200 nm in size.

9. Composition according to any one of the preceding claims, **characterized in that** the acrylic polymer particles range from 20 nm to 150 nm in size.

10. Composition according to any one of the preceding claims, **characterized in that** the acrylic polymer particles range from 50 nm to 100 nm in size.

11. Mascara composition according to any one of the preceding claims, **characterized in that** the acrylic polymer results from the polymerization of at least one monomer containing ethylenic unsaturation chosen from α,β-ethylenic carboxylic acids, esters thereof and amides thereof.

12. Mascara composition according to Claim 11, **characterized in that** the α,β-ethylenic unsaturated carboxylic acid is chosen from acrylic acid, methacrylic acid, crotonic acid, maleic acid and itaconic acid.

13. Mascara composition according to Claim 11 or 12, **characterized in that** the α,β-ethylenic unsaturated carboxylic acid is (meth)acrylic acid.

14. Mascara composition according to Claim 13, **characterized in that** the α,β-ethylenic unsaturated carboxylic acid ester is chosen from (meth)acrylic acid esters.

15. Mascara composition according to Claim 14, **characterized in that** the said ester is chosen from alkyl (meth)acrylates with a C₁-C₃₀ alkyl radical, aryl (meth)acrylates with a C₆-C₁₀ aryl radical, and hydroxyalkyl, (meth)acrylates with a C₂-C₆ hydroxyalkyl radical.

16. Mascara composition according to any one of Claims 11 to 15, **characterized in that** the α,β-ethylenic unsaturated carboxylic acid ester is chosen from methyl methacrylate, ethyl methacrylate, butyl methacrylate, isobutyl methacrylate, 2-ethylhexyl methacrylate, lauryl methacrylate, cyclohexyl methacrylate, hydroxyethyl acrylate, 2-hydroxypropyl acrylate, hydroxyethyl methacrylate, 2-hydroxypropyl methacrylate, benzyl acrylate and phenyl acrylate, and mixtures thereof.

17. Mascara composition according to any one of Claims 11 to 16, **characterized in that** the α,β-ethylenic unsaturated carboxylic acid amide is chosen from N-(C₂-C₁₂)alkyl(meth)acrylamides such as N-ethylacrylamide, N-t-butylacrylamide, N-t-octylacrylamide and N-undecylacrylamide.

18. Mascara composition according to any one of Claims 11 to 17, **characterized in that** the acrylic film-forming polymer also results from the polymerization of at least one styrene monomer such as styrene or α-methylstyrene.

19. Mascara composition according to any one of the preceding claims, **characterized in that** the acrylic polymer is present in a content, as weight of solids, ranging from 5% to 60% by weight relative to the total weight of the composition.

20. Mascara composition according to any one of the preceding claims, **characterized in that** the acrylic polymer is present in a content, as weight of solids, ranging from 10% to 45% by weight relative to the total weight of the composition.

21. Mascara composition according to any one of the preceding claims, **characterized in that** the acrylic polymer is present in a content, as weight of solids, ranging from 15% to 35% by weight relative to the total weight of the composition.

22. Mascara composition according to any one of the preceding claims, **characterized in that** the viscosity of the composition ranges from 5 Pa.s to 15 Pa.s and better still from 6 Pa.s to 12 Pa.s.

23. Mascara composition according to any one of the preceding claims, **characterized in that** the composition comprises at least one thickener.

24. Mascara composition according to any one of the preceding claims, **characterized in that** the composition comprises at least one thickener chosen from the group formed by cellulose-based thickeners, guar gums, xanthan gum, carob gum, scleroglucan gum, gellan gum, rhamsan gum, karaya gum, alginates, maltodextrin, starch and its derivatives, hyaluronic acid and its salts, clays, crosslinked polyacrylic acids, polyglyceryl (meth)acrylates, polyvinylpyrrolidone, polyvinyl alcohol, crosslinked acrylamide polymers and copolymers, crosslinked methacryloyloxyethyltrimethylammonium chloride homopolymers, and associative polymers.

25. Mascara composition according to any one of the preceding claims, **characterized in that** the composition also comprises at least one ingredient chosen from the group formed by plasticizers, coalescers, fillers, dyestuffs, surfactants, preserving agents, oils and cosmetic agents.

26. Mascara composition according to any one of the preceding claims, **characterized in that** the composition also comprises at least one filler chosen from the group formed by fumed silica, starch, talc and polytetrafluoroethylene.

27. Mascara composition according to Claim 25 or 26, **characterized in that** the filler is present in a content ranging from 0.1% to 6% by weight relative to the total weight of the composition.

28. Mascara composition according to any one of the preceding claims, **characterized in that** the composition also comprises an auxiliary film-forming polymer.

29. Mascara product comprising a reservoir (3) containing a mascara composition, especially for the eyelashes, a removable closure means (12) for closing the reservoir and a member (10) for applying the composition to the said fibres, **characterized in that** the mascara composition is in accordance with any one of the preceding claims.

30. Mascara product according to Claim 29, **characterized in that** the applicator member is integral with the removable closure means (12).

31. Mascara product according to either of Claims 29 and 30, **characterized in that** the applicator member (10) is a mascara brush.

32. Mascara product according to any one of Claims 29 to 31, **characterized in that** it also comprises a draining member (7).

33. Make-up product for the eyelashes and/or the eyebrows, **characterized in that** a mascara composition according to any one of Claims 1 to 28 is applied to the eyelashes and/or the eyebrows.

34. Use of a make-up composition according to any one of Claims 1 to 28, to obtain a make-up result on the eyelashes and/or the eyebrows that stays on for more than one day, especially more than two days, and/or a natural make-up result.

35. Use of a film-forming acrylic polymer in the form of solid particles dispersed in the aqueous medium, the said film-forming acrylic polymer being capable of forming a film having a water uptake, after immersion for 10 minutes in water at 25°C, of less than 25% by weight, in a wax-free mascara composition having a viscosity, measured at 25°C, at a shear rate of 200 s⁻¹, ranging from 4 Pa.s to 18 Pa.s, to obtain a make-up result on the eyelashes or the eyebrows that stays on for more than one day, especially more than two days, and/or a natural make-up result.

## Patentansprüche

1. Wachsfreie Mascarazusammensetzung, die in einem kosmetisch akzeptablen wässrigen Medium ein filmbildendes Acrylpolymer in Form von festen, in dem wässrigen Medium dispergierten Partikeln enthält, wobei das filmbildende Acrylpolymer befähigt ist, einen Film zu bilden, der nach 10-minütigem Eintauchen in Wasser von 25 °C eine Wasseraufnahme unter 25 Gew.-% besitzt und wobei die Zusammensetzung eine bei 25 °C und einer Schergeschwindigkeit von 200 s⁻¹ gemessene Viskosität von 4 bis 18 Pa.s besitzt.

2. Mascarazusammensetzung nach Anspruch 1, **dadurch gekennzeichnet, dass** das Acrylpolymer befähigt ist, einen Film mit einer Wasseraufnahme unter 20 Gew.-% zu bilden.

3. Mascarazusammensetzung nach Anspruch 1 oder 2, **dadurch gekennzeichnet, dass** das Acrylpolymer befähigt ist, einen Film mit einer Wasseraufnahme unter 15 Gew.-% zu bilden.

4. Mascarazusammensetzung nach einem der vorhergehenden Ansprüche, **dadurch gekennzeichnet, dass** das Acrylpolymer befähigt ist, einen Film mit einer nach der Norm NF-T-30-016 mit einem Pendelschlagwerk nach Persoz gemessenen Härte von 8 bis 40 Sekunden zu bilden.

5. Mascarazusammensetzung nach Anspruch 4, **dadurch gekennzeichnet, dass** das Acrylpolymer einen Film mit einer Härte von 10 bis 35 Sekunden bilden kann.

6. Zusammensetzung nach Anspruch 4 oder 5, **dadurch gekennzeichnet, dass** das Acrylpolymer einen Film mit einer Härte von 10 bis 30 Sekunden bilden kann.

7. Zusammensetzung nach einem der Ansprüche 4 bis 6, **dadurch gekennzeichnet, dass** das Acrylpolymer befähigt ist, einen Film mit einer Härte von 15 bis 30 Sekunden zu bilden.

8. Zusammensetzung nach einem der vorhergehenden Ansprüche, **dadurch gekennzeichnet, dass** die Partikel des Acrylpolymers eine Größe von 10 bis 200 nm besitzen.

9. Zusammensetzung nach einem der vorhergehenden Ansprüche, **dadurch gekennzeichnet, dass** die Partikel des Acrylpolymers eine Größe von 20 bis 150 nm aufweisen.

10. Zusammensetzung nach einem der vorhergehenden Ansprüche, **dadurch gekennzeichnet, dass** die Partikel des Acrylpolymers eine Größe von 50 bis 100 nm aufweisen.

11. Mascarazusammensetzung nach einem der vorhergehenden Ansprüche, **dadurch gekennzeichnet, dass** das Acrylpolymer bei der Polymerisation mindestens eines Monomers mit ethylenisch ungesättigter Bindung erhalten wird, das unter den α,β-ethylenisch ungesättigten Carbonsäuren, deren Estern und deren Amiden ausgewählt ist.

12. Mascarazusammensetzung nach Anspruch 11, **dadurch gekennzeichnet, dass** die α,β-ethylenisch ungesättigte Carbonsäure unter Acrylsäure, Methacrylsäure, Crotonsäure, Maleinsäure und Itaconsäure ausgewählt ist.

13. Mascarazusammensetzung nach Anspruch 11 oder 12, **dadurch gekennzeichnet, dass** die α,β-ethylenisch ungesättigte Carbonsäure die (Meth)acrylsäure ist.

14. Mascarazusammensetzung nach Anspruch 13, **dadurch gekennzeichnet, dass** der Ester der α,β-ethylenisch ungesättigten Carbonsäure unter den Estern von (Meth)acrylsäure ausgewählt ist.

15. Mascarazusammensetzung nach Anspruch 14, **dadurch gekennzeichnet, dass** der Ester unter den Alkyl(meth)acrylaten mit C₁₋₃₀-Alkylgruppe, Aryl(meth)acrylaten mit C₈₋₁₀-Arylgruppe und Hydroxyalkyl(meth)acrylaten mit C₂₋₆-Hydroxyalkylgruppe ausgewählt ist.

16. Mascarazusammensetzung nach einem der Ansprüche 11 bis 15, **dadurch gekennzeichnet, dass** der Ester der α,β-ethylenisch ungesättigten Carbonsäure unter Methylmethacrylat, Ethylmethacrylat, Butylmethacrylat, Isobutylacrylat, 2-Ethylhexylmethycrylat, Laurylmethacrylat, Cyclohexylmethacrylat, Hydroxyethylacrylat, 2-Hydroxypropylacrylat, Hydroxyethylmethacrylat, 2-Hydroxypropylmethacrylat, Benzylacrylat, Phenylacrylat und deren Gemischen ausgewählt ist.

17. Mascarazusammensetzung nach einem der Ansprüche 11 bis 16, **dadurch gekennzeichnet, dass** das Amid der α,β-ethylenisch ungesättigten Carbonsäure unter den N-Alkyl(C₂₋₁₂)-(meth)-acrylamiden, wie N-Ethylacrylamid, N-*t*-Butylacrylamid, N-*t* Octylacrylamid und N-Undecylacrylamid, ausgewählt ist.

18. Mascarazusammensetzung nach einem der Ansprüche 11 bis 17, **dadurch gekennzeichnet, dass** das filmbildende Acrylpolymer auch bei der Polymerisation mindestens eines Styrolmonomers, wie Styrol oder alpha-Methylstyrol, gebildet wird.

19. Mascarazusammensetzung nach einem der vorhergehenden Ansprüche, **dadurch gekennzeichnet, dass** das Acrylpolymer in einer als Trockensubstanz ausgedrückten Gewichtsmenge von 5 bis 60 Gew.-%, bezogen auf das Gesamtgewicht der Zusammensetzung, enthalten ist.

20. Mascarazusammensetzung nach einem der vorhergehenden Ansprüche, **dadurch gekennzeichnet, dass** das Acrylpolymer in einer als Trockensubstanz ausgedrückten Gewichtsmenge von 10 bis 45 Gew.-%, bezogen auf das Gesamtgewicht der Zusammensetzung, enthalten ist.

21. Mascarazusammensetzung nach einem der vorhergehenden Ansprüche, **dadurch gekennzeichnet, dass** das Acrylpolymer in einer als Trockensubstanz ausgedrückten Gewichtsmenge von 15 bis 35 Gew.-%, bezogen auf das Gesamtgewicht der zusammensetzung, enthalten ist.

22. Mascarazusammensetzung nach einem der vorhergehenden Ansprüche, **dadurch gekennzeichnet, dass** die Viskosität der Zusammensetzung im Bereich von 5 bis 15 Pa.s und besser 6 bis 12 Pa·s liegt.

23. Mascarazusammensetzung nach einem der vorhergehenden Ansprüche, **dadurch gekennzeichnet, dass** die Zusammensetzung mindestens ein Verdickungsmittel enthält.

24. Mascarazusammensetzung nach einem der vorhergehenden Ansprüche, **dadurch gekennzeichnet, dass** die Zusammensetzung mindestens eine Verdickungsmittel enthält, das unter den Verdickungsmitteln auf Cellulosebasis, Guargummen, Xanthangummi, Johannisbrotkernmehl, Skleroglucanen, Gellangumi, Rhamsangummi, Karaya-Gummi, Alginaten, Maltodextrin, Stärke und Stärkederivaten, Hyaluronsäure und ihren Salzen, Tonen, vernetzten Polyacrylsäuren, Polyglyceryl(meth)acrylaten, Polyvinylpyrrolidon, Polyvinylalkohol, vernetzten Acrylamidpolymeren und -copolymeren, vernetzten Methacryloyloxyethyltrimethylammoniumchlorid-Homopolymeren und assoziativen Polymeren ausgewählt ist.

25. Mascarazusammensetzung nach einem der vorhergehenden Ansprüche, **dadurch gekennzeichnet, dass** die Zusammensetzung mindestens einen Bestandteil enthält, der unter den Weichmachern, Koaleszenzmitteln, Füllstoffen, Farbmitteln, grenzflächenaktiven Stoffen, Konservierungsmitteln, Ölen und kosmetischen Wirkstoffen ausgewählt ist.

26. Mascarazusammensetzung nach einem der vorhergehenden Ansprüche, **dadurch gekennzeichnet, dass** die Zusammensetzung mindestens einen Füllstoff enthält, der unter pyrogener Kieselsäure, Stärke, Talk und Polytetrafluorethylen ausgewählt ist.

27. Mascarazusammensetzung nach Anspruch 25 oder 26, **dadurch gekennzeichnet, dass** der Füllstoff in einer Menge von 0,1 bis 6 Gew.-%, bezogen auf das Gesamtgewicht der Zusammensetzung, enthalten ist.

28. Mascarazusammensetzung nach einem der vorhergehenden Ansprüche, **dadurch gekennzeichnet, dass** die Zusammensetzung ferner ein zusätzliches filmbildendes Polymer enthält.

29. Mascaraprodukt, das einen Behälter (3) mit einer Mascarazusammensetzung, insbesondere für die Wimpern, eine abnehmbare Einrichtung zum Verschließen (12), die dazu vorgesehen ist, den Behälter zu verschließen, und ein Werkzeug zum Auftragen (10) der Zusammensetzung auf die Fasern enthält, **dadurch gekennzeichnet, dass** die Mascarazusammensetzung eine Zusammensetzung nach einem der vorhergehenden Ansprüche ist.

30. Mascaraprodukt nach Anspruch 29, **dadurch gekennzeichnet, dass** das Werkzeug zum Auftragen fest mit der abnehmbaren Einrichtung zum Verschließen (12) verbunden ist.

31. Mascaraprodukt nach einem der Ansprüche 29 oder 30, **dadurch gekennzeichnet, dass** das Werkzeug zum Auftragen (10) eine Mascarabürste ist.

32. Mascaraprodukt nach einem der Ansprüche 29 bis 31, **dadurch gekennzeichnet, dass** es ferner einen Abstreifer (7) umfasst.

33. Verfahren zum Schminken der Wimpern und/oder Augenbrauen, **dadurch gekennzeichnet, dass** auf die Wimpern und/oder Augenbrauen eine Mascarazusammensetzung nach einem der Ansprüche 1 bis 28 aufgetragen wird.

34. Verwendung einer Zusammensetzung zum Schminken nach einem der Ansprüche 1 bis 28 für die Bildung einer Schminke auf den Wimpern und/oder Augenbrauen, die eine Haltbarkeit größer als einen Tag und insbesondere größer als 2 Tage aufweist, und/ oder einer natürlichen Schminke.

35. Verwendung eines filmbildenden Acrylpolymers in Form von festen, in dem wässrigen Medium dispergierten Partikeln, wobei das filmbildende Acrylpolymer befähigt ist, nach 10-minütigem Eintauchen in Wasser von 25 °C einen Film mit einer Wasseraufnahme unter 25 Gew.-% zu bilden, in einer Mascarazusammensetzung, die kein Wachs enthält und eine bei 25 °C und einer Schergeschwindigkeit von 200 s⁻¹ gemessene Viskosität von 4 bis 18 Pa·s aufweist, zur Bildung einer Schminke auf den Wimpern oder Augenbrauen, die eine Haltbarkeit größer als einen Tag und insbesondere größer als zwei Tage aufweist, und/oder einer natürlichen Schminke.
